# EUROPEAN PATENT SPECIFICATION

(11) **EP 2 452 674 B1**
(45) Date of publication and mention of the grant of the patent: **26.03.2014**
(21) Application number: 11190964.4
(22) Date of filing: 08.08.2007
(51) Int. Cl.: A61K 31/496, A61K 31/4545

(54) **Compositions and methods for increasing blood platelet levels in humans**
Zusammensetzungen und Verfahren zur Erhöhung der Blutplättchenanteile bei Menschen
Compositions et procédés pour augmenter les niveaux de plaquettes dans le sang chez les êtres humains

(30) Priority: 08.08.2006 US 836334 P
(43) Date of publication of application: 16.05.2012
(62) Divisional of application: 07811274.5
(73) Proprietor: Akarx, Inc., Woodcliff Lake, NJ (US)
(72) Inventor: Desjardins, Robert E., Allendale, NJ 07924 (US); Lucek, Rudolph, Bernardsville, NJ 07924 (US)
(74) Representative: HOFFMANN EITLE

(56) References cited:
- DESJARDINS ROBERT E ET AL: "Single and multiple oral doses of AKR-501 (YM477) increase the platelet count in healthy volunteers.", BLOOD, vol. 108, no. 11, Part 1, November 2006 (2006-11), page 145A, XP002539262, & 48TH ANNUAL MEETING OF THE AMERICAN-SOCIETY-OF-HEMATOLOGY; ORLANDO, FL, USA; DECEMBER 09 -12, 2006 ISSN: 0006-4971

## Description

### Field of the Invention

The present invention is directed to compositions for increasing blood platelet levels in humans.

### Background of the Invention

Thrombocytopenia is a potentially serious condition characterized by a deficiency of platelets in the circulatory system. It is associated with an increased risk of bleeding particularly from small capillaries resulting in thrombocytopenic purpura. The causes of thrombocytopenia are heterogeneous and include decreases in platelet production in the bone marrow and decreases in platelet survival in the blood. There are specific disease related thrombocytopenias such as Idiopathic Thrombocytopenic Purpura and thrombocytopenias caused by the indirect effect of other diseases on the bone marrow including malignancies and infections such as hepatitis. Currently, management of thrombocytopenia is primarily based on platelet transfusion. Despite their effectiveness, approximately 30% of platelet transfusions are associated with serious complications including alloimmunization, febrile and allergic reactions, circulatory overload, acute pulmonary injury and bacterial or viral infections. In the 15 to 25% of patients who require repeated platelet transfusions, the platelet response is inadequate due to human leukocyte antigen (HLA) alloimmunization. Therefore, a safe thrombopoietic agent that can lessen or eliminate the need for platelet transfusion would benefit patient health and could significantly lower health-care costs.

Thrombopoietin (TPO) is the principal physiologic regulator of platelet production. TPO is produced constitutively in liver and other organs, circulates in the bloodstream, and is delivered to bone marrow, where it stimulates the early development of multiple hematopoietic lineages and megakaryocytopoiesis. TPO exerts its effect on megakaryocytopoiesis and thrombopoiesis through binding to and activation of the cytokine receptor, c-Mpl, which is express on hematopoietic stem sells (HSCs), on cells of the megakaryocytic lineage, and platelets.

The compound of Formula I: 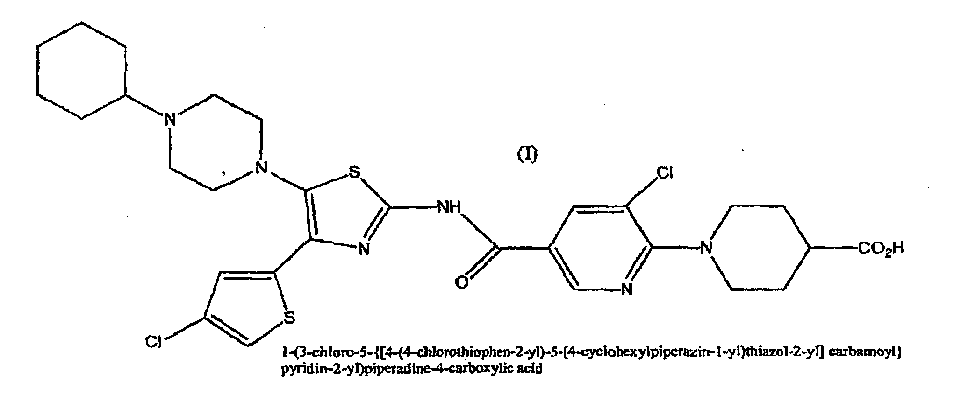 is an orally administered, small molecule c-Mpl agonist that mimics the biological effects on thrombopoietin. This compound is disclosed in WO03/062233 (Example 16) and WO04/029049 (as the maleic acid salt), which are incorporated by reference in their entireties. The chemical name for Formula I is 1-(3-chloro-5-{[4-(4-chlorothiophen-2-yl)-5-(4-cyclohexylpiperazin-1-yl)thiazol-2-yl] carbamoyl} pyridin-2-yl)piperadine-4-carboxylic acid.

There exists a need in the art for formulations of Formula I which provide pharmacokinetic parameters useful for the treatment and/or prevention of thrombocytopenia.

### Summary of the Invention

It is an object of the present invention to provide formulations of 1-(3-chloro-5-{[4-(4-chlorothiophen-2-yl)-5-(4-cyclohexylpiperazin-1-yl)thiazol-2-yl] carbamoyl} pyridin-2-yl)piperadine-4-carboxylic acid (hereinafter "the drug of Formula I") which provide pharmacokinetic parameters useful for increasing the level of blood platelets after administration to a human subject

The present invention is directed to an immediate release oral pharmaceutical dosage form comprising a pharmaceutically acceptable excipient and 10 to 75 mg of the drug of Formula I or a pharmaceutically acceptable salt thereof, to increaseplatelet levels in humans, the dosage form providing a mean maximum plasma concentration (Cₘₐₓ) of from about 3 ng/ml to about 8 ng/ml for each 1 mg of the drug included in the dosage form, after single dose administration to human subjects.

In order that the invention described herein may be more fully understood, the following definitions are provided for the purposes of the disclosure:

The term "provide a increase in platelet levels" shall mean an increase in a subject's or patient's platelets from baseline that is at least about 25%; preferably at least about 50%; more preferably greater than 50%.

The term "drug of Formula I" shall mean 1-(3-chloro-5-{[4-(4-chlorothiophen-2-yl)-5-(4-cyclohexylpiperazin-1-yl)thiazol-2-yl] carbamoyl} pyridin-2-yl)piperadine-4-carboxylic acid and any pharmaceutically acceptable salt, hydrate, solvate, polymorph, metabolite, derivative, pro-drug, free-base or any combination thereof.

The term "human subject" shall mean a normal healthy male or female volunteer and/or any individual that presents with clinical signs and symptoms of thrombocytopenia or any disease or disorder that may cause thrombocytopenia.

The term "thrombocytopenia" shall mean any and all disease related thrombocytopenias, which include, but are not limited to, idiopathic thrombocytopenic purpura (ITP), thrombotic thrombocytopenic purpura (TTP), hemolytic-uremic syndrome (HUS), disseminated intravascular coagulation (DIC), paroxysmal nocturnal hemoglobinuria (PNH), antiphospholipid syndrome, systemic lupus erythematosus (SLE), post transfusion purpura, neonatal alloimmune thrombocytopenia (NAITP), splenic sequestration of platelets due to hypersplenism, dengue fever, thrombocytopenia of myelodysplastic syndromes (MDS), and chemotherapy-induced thrombocytopenia) either as a substitute for, or in combination with, platelet transfusion, Hepatitis C. The term "thrombocytopenia" also shall mean other disease processes that can cause decreased platelets such as, but are not limited to vitamin B12 or folic acid deficiency; leukemia; decreased production of thrombopoietin by the liver in liver failure; sepsis, systemic viral or bacterial infection; hereditary syndromes including, but not limited to, Congenital Amegakaryocytic Thrombocytopenia (CAMT), thrombocytopenia absent radius syndrome, Fanconi anemia, Bernard-Soulier syndrome, May-Hegglin anomaly; Grey platelet syndrome; and Alport syndrome.

The expression "bioequivalent" or "bioequivalence" is a term of art and is intended to be defined in accordance with Approved Drug Products with Therapeutic Equivalence Evaluations, 15th Edition, pages vii-xvii, which is published by the U.S. Department of Health and Human Services, and is commonly known as the "Orange Book". Bioequivalence of different formulations of the same drug substance involves equivalence with respect to the rate and extent of drug absorption. The extent and rate of absorption of the test formulation is compared to a reference formulation in order to determine whether the two formulations are bioequivalent. The standard bioequivalence study is conducted in crossover fashion by extensive testing which includes administering single doses of the test and reference drugs to a number of volunteers, usually 12 to 24 healthy normal adults, and then measuring the blood or plasma levels of the drug over time. Detailed guidelines for establishing the bioequivalence of a formulation with a reference formulation have been published by the FDA Office of Generic Drugs, Division of Bioequivalence.

The mean pharmacokinetic ranges set forth in the present application and in the appended claims are meant to encompass ranges that would be considered by those skilled in the art to be "bioequivalent." In this regard, in other words, a particular pharmacokinetic property of the oral formulations of the invention are deemed to encompass formulations of 1-(3-chloro-5-{[4-(4-chlorothiophen-2-yl)-5-(4-cyclohexylpiperazin-1-yl)thiazol-2-yl] carbamoyl} pyridin-2-yl)piperadine-4-carboxylic acid (Formula 1) that could be rated an AB therapeutic equivalent based on the referenced pharmacokinetic parameter., e.g., if the product in question is the subject of an Abbreviated New Drug Application (ANDA) filed under section 505(j) of the Food Drug and Cosmetic Act (FDCA) (21 U.S.C. 355(j)) which contains a bioequivalence study to a reference drug encompassed by the appended claims. The pharmacokinetic characteristics of the concentration-time curve, such as the maximum observed plasma concentration (Cmax), the time to reach Cmax, and the area under the plasma concentration versus time curve (AUC), are examined by statistical procedures which are well-established in the field of pharmacokinetics. Two formulations whose rate and extent of absorption differ by - 20%/+25% or less are generally considered to be bioequivalent, and such formulations are deemed to be within the scope of the appealed claims.

### Brief Description of the Figures

Figure 1 depicts a graph of the mean maximum change from baseline platelet count from Example 1.

Figure 2 depicts a graph of the mean maximum change from baseline platelet count from Example 2.

Figure 3 depicts a graph of the average concentration profiles of the three doses of drug administered in Example 3.

### Detailed Description

Pharmacology studies demonstrate that the compound of Formula I: 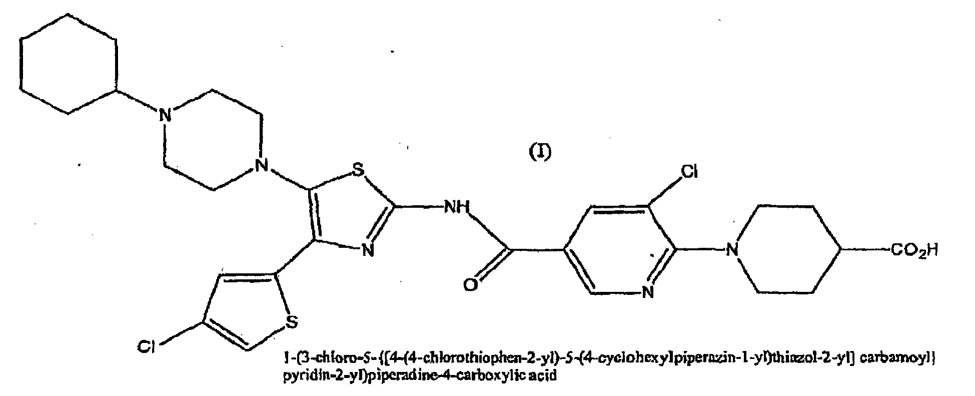 stimulates the proliferation of cultured human c-Mpl-Ba/F3 cells and promotes the differentiation of human cord blood (CB) CD34⁺ cells to human megakaryocytes in a concentration-dependent manner and having a maximum effect similar to that of recombinant human TPO (rhTPO). The compound of Formula I did not promote the differentiation of human hematopoietic progenitor cells, other than those of megakaryocytic lineage.

The compound of Formula I does not interfere with rhTPO binding to human platelets at concentrations up to 100µM suggesting that the site of action of Formula Ion human c-Mpl differs from that of rhTPO. It is highly species specific, showing activity only in the chimpanzee and man. While the compound of Formula I enhances human platelet proliferation and differentiation, it does not appear to effect platelet function as measured by platelet aggregation and activation.

After single intravenous administration of drug of Formula I as the monomaleate salt to mice and dogs, plasma concentrations of Formula I decreased in a biphasic manner. The terminal elimination half-life (t_{1/2}) was 2.7 hours in both species. Following oral administration, Formula I is well absorbed having an absolute bioavailability of 91 % in the mouse and 52% in the dog.

The *in-vitro* plasma protein binding of drug of Formula I was independent of concentration over the concentration range of 0.05 to 50 µg/mL and was 87.2 - 89.1% in mice; 87.6 - 89.2 % in rats; 88.3 - 92.0 % in rabbits; 97.3 - 97.9.6% in dogs; 97.0 - 97.6 in monkeys; and 96.3 - 96.6% in humans.

The drug of Formula I does not appear to be metabolized by mouse, dog or human liver microsomal enzymes and did not inhibit the major human liver cytochrome P450 isoenzymes (CYP1A2, 2C9, 2C19, 2D6 and 3A4). Drug of Formula I does not appear to possess a liability for drug-drug interactions due to metabolic inhibition of cytochrome P450 liver enzymes.

Following repeat dosing for 4 weeks in rats (3, 10, 30, 100mg/kg/day) and cynomolgus monkey (3, 10, 100, 1000mg/kg/day), changes in the gastric mucosa were characterized in the rat by degeneration of chief cells and parietal cells and in cynomolgus monkey by necrosis of the surface epithelium and atrophy of the fundic glands and edema and inflammatory infiltrates in the lamia propria of the fundus. These changes resolved on drug withdrawal in the rat but not entirely in the high dose monkeys.

In the rat and cynomolgus monkey, muscle degeneration/necrosis was observed in femoral, lower thigh or multifides muscles in 1 or 2 rats per dose group and in one or more monkeys in all dose groups. Elevated levels of CK and aldolase were also observed. In the monkey, the presence of elevated CK and aldolase levels prior to the administration of the first dose of drug of Formula I as the monomaleate salt suggested that many of the monkeys had skeletal muscle changes before ever receiving Formula I. The presence of a skeletal muscle lesion in a control monkey in the recovery study suggests that not all lesions described in the study were related to the administration of drug of Formula I.

In the dog following administration of drug of Formula I as the monomaleate salt (10, 30, 100, 300 mg/kg/day) for 4 weeks, microscopic changes were found in the cortex of the kidney, most notably epithelial cell regeneration of proximal tubules and interstitial subacute inflammation. At the end of the recovery period, regeneration of renal tubular epithelial persisted in the majority of the high dose animals (100, 300 mg/kg/day). However, all other renal lesions noted during the dosing period had resolved by the end of the recovery period. No muscle lesions were observed in the dog at any dose of the drug of Formula I.The drug of Formula I administered as the monomaleate salt suppressed the hERG current in a dose dependent manner with an IC₅₀ of approximately 1.4 x 10⁻⁶ mol/L. Formula I monomaleate had no effect on the action potential in isolated guinea pig papillary muscles. No cardiovascular or respiratory system effects were observed in the conscious dog up to a dose of 300 mg/kg. In the cynomolgus monkeys, a prolongation of QT interval was reported at 100 and 1000 mg/kg but this effect appears to be related to a decrease in heart rate resulting from animal acclimation and not drug related.

Disclosed herein are the results from a method of treating thrombocytopenia, comprising orally administering a single daily dose of 1-(3-chloro-5-{[4-(4-chlorothiophen-2yl)-5-(4-cyclohexylpiperazin-1-yl)thiazol-2-yl] carbamoyl} pyridin-2-yl)piperadine-4-carboxylic acid, or a pharmaceutically acceptable salt thereof in an amount from about 1mg to about 100mg to achieve a mean maximum plasma concentration (Cₘₐₓ) of from about 5.7 ng/ml to about 475 ng/ml, Administration of the single daily dose achieves a mean AUC₀₋ₗₐₛₜ of from about 130 ng-hr/ml to about 10864 ng·hr/ml; a mean t_{1/2} of from about 18 to about 24 hours; and a mean time to maximum plasma concentration (Tₘₐₓ) from about 4.7 to about 6.2 are achieved.

When a single 1mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of about 5.7 ng/ml; a mean AUC₀₋ₗₐₛₜ of of about 130 ng·hr/ml; and a mean time to maximum plasma concentration (Tₘₐₓ) of about 6.1 are achieved.

When a single 3mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of from about 14 ng/ml to about 17 ng/ml and a mean AUC₀₋ₗₐₛₜ of from about 235 ng·hr/ml to about 400 ng·hr/ml; and a mean time to maximum plasma concentration (Tₘₐₓ) of about 5.5 are achieved.

When a single 10mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cmax) of from about 53 ng/ml to about 69 ng/ml (± 20%); a mean AUC0-last of from about 840 ng.hr/ml to about 1645 ng·hr/ml; and a mean time to maximum plasma concentration (Tₘₐₓ) from about 4.8 to about 6.0 are achievd.

In certain embodiments, when a single 10mg dose is administered over a period of about fourteen (14) days, a mean maximum plasma concentration (Cₘₐₓ) of about 94 ng/ml; a mean AUC₀₋ₗₐₛₜ of about 2364 ng·hr/ml; and a mean time to maximum plasma concentration (Tₘₐₓ) of about 4.4 are achieved.

When a single 20mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of from about 121 ng/ml to about 1.68 ng/ml; a mean AUC₀₋ₗₐₛₜ of from about 1956 ng.hr/ml to about 3597 ng·hr/ml; and a mean time to maximum plasma concentration (Tₘₐₓ) of about 5.2 are achieved.

In certain embodiments, when a single 20mg dose is administered over a period of about fourteen (14) days, a mean maximum plasma concentration (Cₘₐₓ) of about 204 ng/ml; a mean AUC₀₋ₗₐₛₜ of about 3610 ng.hr/ml; and a mean time to maximum plasma concentration (Tₘₐₓ) of about 5.5 are achieved.

When a single 50mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of about 311 ng/ml; a mean AUC₀₋ₗₐₛₜ of about 6879 ng·hr/ml; and a mean time to maximum plasma concentration (Tₘₐₓ) of about 4.8 are achieved.

When a single 75mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of from about 473 ng/ml; a mean AUC₀₋ₗₐₛₜ of about 10824 ng-hr/ml; and a mean time to maximum plasma concentration (Tₘₐₓ) of about 6.0 are achieved.

When a 100mg daily dose of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof is administered to a human patient in need thereof, a mean maximum plasma concentration (Cₘₐₓ) of about 388 ng/ml; a mean AUC₀₋ₗₐₛₜ of about 10863 ng·hr/ml; and a mean time to maximum plasma concentration (Tₘₐₓ) of about 6.2 are achieved.

Administration of a single dose of from about 1mg to about 100mg of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof to a human patient in need thereof achieves a mean t_{1/2} of from about 18 to about 24 hours.

In certain embodiments, the present invention provides an oral dosage form for treatment of thrombocytopenia, comprising a therapeutically effective amount of 1-(3-chloro-5-{[4-(4-chlorothiophen-2 yl)-5-(4-cyclohexylpiperazin-1-yl)thiazol-2-yl] carbamoyl} pyridin-2-yl)piperadine-4-carboxylic acid, or a pharmaceutically acceptable salt thereof; and at least one pharmaceutically acceptable excipient, wherein said therapeutically effective amount is a single dose ranging from about 10mg to 75mg, said single dose providing an increase in platelet count from baseline in a human patient in need thereof of at least about 25%.

The dosage form of the present invention achieves: i) a mean maximum plasma concentration (Cmax) of from about 5.7 ng/ml to about 475 ng/ml; ii) a mean AUC₀₋ₗₐₛₜ of from about 130 ng·hr/ml to about 10864 ng.hr/ml; and iii) a mean t_{1/2} of from about 18 to about 24 hours, when administered to a human patient in need thereof.

The present invention is directed to an oral pharmaceutical dosage form comprising a pharmaceutically acceptable excipient and 10 to 75mg of the drug of Formula I or a pharmaceutically acceptable salt thereof, to increase platelet levels in humans, the dosage form providing a mean maximum plasma concentration (Cₘₐₓ) of from about 3 ng/ml to about 8 ng/ml for each 1 mg of the drug included in the dosage form, after single dose administration to human subjects.

In certain embodiments, the formulation comprises 10 mg of the drug and provides a mean maximum plasma concentration (Cₘₐₓ) of from about 30 ng/ml to about 80 ng/ml or from about 50 ng/ml to about 75 ng/ml, after single dose administration to human subjects.

In certain embodiments, the formulation comprises 20 mg of the drug and provides a mean maximum plasma concentration (Cₘₐₓ) of from about 60.0 ng/ml to about 160.0 ng/ml or from about 130 ng/ml to about 155 ng/ml, after single dose administration to human subjects.

In certain embodiments, the formulation comprises 50 mg of the drug and provides a mean maximum plasma concentration (Cₘₐₓ) of from about 150.0 ng/ml to about 400 ng/ml or from about 250 ng/ml to about 350 ng/ml, after single dose administration to human subjects.

In certain embodiments, the formulation comprises 75 mg of the drug and provides a mean maximum plasma concentration (Cₘₐₓ) of from about 225 ng/ml to about 600 ng/ml or from about 400 ng/ml to about 500 ng/ml, after single dose administration to human subjects.

In certain embodiments, the present invention is directed to an oral pharmaceutical dosage form comprising a pharmaceutically acceptable excipient and an effective amount of the drug of Formula I or a pharmaceutically acceptable salt thereof, to increase platelet levels in humans, the dosage form providing a mean AUC₀₋₂₄ of from about 50 ng.hr/ml to about 250 ng·hr/ml for each 1 mg of the drug included in the dosage form, after single dose administration to human subjects.

In certain embodiments, the formulation comprises 10 mg of the drug and provides a mean AUC₀₋₂₄ of from about 500 ng.hr/ml to about 2500 ng.hr/ml or from about 1500 ng·hr/ml to about 2400 ng.hr/ml, after single dose administration to human subjects.

In certain embodiments, the formulation comprises 20 mg of the drug and provides a mean AUC₀₋₂₄ of from about 1000 ng.hr/ml to about 5000 ng·hr/ml or from about 3500 ng·hr/ml to about 4500 ng·hr/ml, after single dose administration to human subjects.

In certain embodiments, the formulation comprises 50 mg of the drug and provides a mean AUC₀₋₂₄ of from about 2500 ng·hr/ml to about 12500 ng.hr/ml or from about 8000 ng·hr/ml to about 10000 ng·hr/ml, after single dose administration to human subjects.

In certain embodiments, the formulation comprises 75 mg of the drug and provides a mean AUC₀₋₂₄ of from about 3750 ng.hr/ml to about 18750 ng·hr/ml or from about 300 ng·hr/ml to about 635 ng.hr/ml, after single dose administration to human subjects.

In certain embodiments, the present invention is directed to an oral pharmaceutical dosage form comprising a pharmaceutically acceptable excipient and an effective amount of the drug of Formula I or a pharmaceutically acceptable salt thereof, to increase platelet levels in humans, the dosage form providing an elimination half-life (T_{1/2}) of from about 10 hours to about 25 hours, from about 12 hours to about 18 hours or from about 14 hours to about 16 hours, after single dose administration to human subjects.

In certain embodiments, the present invention is directed to formulations and methods as disclosed herein, wherein the mean change in platelet count from baseline is increased by at least about 25%, at least about 50%, at least about 75%, at least about 100% or at least about 150%; or from about 25% to about 200% or from about 75% to about 150%. In certain embodiments, the platelet count is increased from baseline by greater than 50%.

In the present invention, the drug of Formula I may be in the form of the free base, a pharmaceutically acceptable salt, hydrate, solvate, polymorph, metabolite, derivative or any combination of the foregoing.

Pharmaceutically acceptable salts may include, but are not limited to, mineral acids such as hydrochloric acid, hydrobromic acid, hydroiodic acid, sulfuric acid, nitric acid, phosphoric acid, and the like; an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfanic acid, p-toluenesulfonic acid, aspartic acid, glutamic acid, and the like; salts with an inorganic base such as sodium, potassium, magnesium, calcium, and the like; salts with an organic base such as methylamine, ethylamine, ethanolamine, lysine, omithine, and the like; and ammonium salts, and the like.

In certain embodiments, the drug of Formula I is in the form of the monomaleate salt.

### FORMULATIONS

The drug of Formula I is contained together with pharmaceutically acceptable excipients in an immediate release oral dosage form. The immediate release dosage forms of the present invention include, but are not limited to, tablets, soft or hard gelatin capsules, solutions or suspensions.

Pharmaceutical compositions and dosage forms of the invention preferably contain one or more pharmaceutically acceptable excipients. Such excipients include, but are not limited to, carriers, diluents, fillers, lubricants and glidants.

Carriers, diluents and fillers suitable for use in pharmaceutical compositions and dosage forms include, but are not limited to, calcium carbonate, calcium phosphate, dibasic calcium phosphate, tribasic calcium sulfate, calcium carboxymethylcellulose, cellulose, cellulose (e.g., microcrystalline cellulose, silicified microcrystalline cellulose, and cellulose acetate), dextrates, dextrin, dextrose (glucose), fructose, lactitol, lactose, magnesium carbonate, magnesium oxide, matitol, maltodextrins, maltose, sorbitol, starch (e.g., pregelatinized starch), sucrose, sugar, and xylitol.

Lubricants that can be used in pharmaceutical compositions and dosage forms of the invention include, but are not limited to, agar, calcium stearate, ethyl oleate, ethyl laureate, glycerin, glyceryl palmitostearate, hydrogenated vegetable oil (e.g., corn oil, cottonseed oil, olive oil, peanut oil, sesame oil, soybean oil, and sunflower oil), magnesium oxide, magnesium stearate, mannitol, poloxamer, glycols (e.g., polyethylene glycol), sodium benzoate, sodium lauryl sulfate, sodium stearyl, sorbitol, stearic acid, talc, zinc stearate, and mixtures thereof.

Glidants include, for example, coagulated aerosols of synthetic silica colliodal silicon dioxide, magnesium trisilicate, powdered cellulose, pyrogenic silicon dioxide products (e.g., CAB-O-SIL sold by Cabot Co. of Boston, Mass.), starch, syloid silica gels (e.g., AEROSIL 200, manufactured by W.R. Grace Co. of Baltimore, Md.), talc, tribasic calcium phosphate, and mixtures thereof. If used, lubricants are typically used in an amount of less than about 1 weight percent of the pharmaceutical compositions or dosage forms into which they are incorporated.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form the compositions of the invention. The amount of disintegrant used varies based upon the type of formulation, and is readily discernible to those of ordinary skill in the art. Disintegrants that can be used in pharmaceutical compositions and dosage forms of the invention include, but are not limited to, agar-agar, algins (e.g., alginic acid), calcium carbonate, carboxmethylcellulose, cellulose (e.g., hydroxypropyl cellulose, microcrystalline cellulose, and silicified microcrystalline cellulose), clays, colloid silicon dioxide, croscarmellose sodium, crospovidone, gums, magnesuim aluminium silicate, methylcellulose, polacrilin potassium, sodium alginate, sodium starch glycolate, starch (e.g., pregelatinized starch, potato starch, and tapioca starch), and mixtures thereof.

Pharmaceutical compositions and dosage forms can also contain wetting, emulsifying, and pH buffering agents.

Other suitable fillers, binders, disintegrates, lubricants and additional excipients which may be used are described in Handbook of Pharmaceutical Excipients, 2nd Edition, American Pharmaceutical Association; The Theory and Practice of Industrial Pharmacy, 2nd Edition, Lachman, Leon, 1976; Pharmaceutical Dosage Forms: Tablets Volume 1, 2.sup.nd Edition, Lieberman, Hebert A., et al, 1989; Modem Pharmaceutics, Banker, Gilbert and Rhodes, Christopher T, 1979; and Remington's Pharmaceutical Sciences, 20th Edition, 2000.

Pharmaceutical compositions of the invention suitable for administration can be presented as discrete dosage forums, such as capsules (e.g., gelcaps), caplets, tablets, troches, lozenges, dispersions, and suppositories each containing a predetermined amount of an active ingredient as a powder or in granules, a solution, or a suspension in an aqueous or non-aqueous liquid, an oil-in-water emulsion, or a water-in-oil liquid emulsion.

Dosage forms of the present invention can be prepared by any of the methods of pharmacy, but all methods include the step of bringing the active ingredient into association with the excipient, which constitutes one or more necessary ingredients. In general, the compositions are prepared by uniformly admixing the active ingredient with liquid excipients or finely divided solid excipients or both, and then, if necessary, shaping the product into the desired presentation. If desired, tablets can be coated by standard aqueous or nonaqueous techniques.

The pharmaceutical composition of the invention may be prepared, using standard techniques and manufacturing processes generally known in the art, for example by dry blending the components. For example, the active agent, one or more fillers, one or more optional excipients (e.g., binders and/or disintegrants, as well as other additional optional excipients) are blended together. The components of the blend prior to blending, or the blend itself, may be passed through a mesh screen, for example a 400-700 um mesh screen. A lubricant, which may also be screened, is then added to the blend and blending continued until a homogeneous mixture is obtained. The mixture is then compressed into tablets. Alternatively, a wet granulation technique can be employed. For example, the active agent and excipient(s) are blended together, for example by using a granulator, and the powder blend is granulated with a small volume of purified water. The granulate is dried and passed though a mill. The remainder of the disintegrant and a lubricant are added to the milled granulation and after blending the resultant homogeneous mixture is compressed into tablets. It will be appreciated that modifications of the dry blending and wet granulation techniques, including the order of addition of the components and their screening and blending prior to compression into tablets, may be carried out according to principles well known in the art.

A tablet coating may then be applied, for example by spray-coating. With a water-based film coating formulation. The coating may comprise, for example, lactose, hydroxypropyl methylcellulose, triacetin, titanium dioxide and ferric oxides.

In certain embodiments, the drug of Formula I is contained in a liquid oral dosage form such an elixir, emulsion, syrup, solution or suspension. Initially, drug of Formula I may be dissolved prior to incorporation into a liquid dosage form by utilizing any pharmaceutically acceptable solvent. Suitable solvents for use in the present invention may include, but are not limited to, alcohol, glycerin, propylene glycol, water (purified or sterile) and the like.

The liquid dosage forms of the present invention may contain any pharmaceutically acceptable excipient suitable for use in liquid dosage forms. For example, the liquid dosage forms of the present invention may optionally contain one or more antioxidants, if necessary, taste modifiers, flavors or flavoring agents, sweeteners, glidants, suspending agents, anti-caking agents, emulsifying agents, buffering agents, and preservatives.

Suitable suspending agents include, but are not limited to, sodium and calcium carboxymethylcelluloses, microcrystalline cellulose, xanthan gum, carrageenan and any combinations and mixtures thereof.

Suitable sweeteners may be any convenient agent(s) known in the art for this purpose and may be selected from any compatible sweetener groups such as natural sweeteners like sucrose, fructose, glucose, dextrose, xylitol, sorbitol, or manitol, as well as artificial sweeteners such as aspartame, saccharin, acesulfame K and sucrolose.

Suitable flavors and flavoring agents may include, but are not limited to, synthetic flavor oils and flavoring aromatics and/or natural oils, extracts from plant leaves, flowers, fruits, and so forth and combinations thereof. Suitable oils include, for example, cinnamon oil, oil of wintergreen, peppermint oils, clove oil, bay oil, anise oil, eucalyptus oil, thyme oil, cedar leaf oil, oil of nutmeg, oil of sage, oil of bitter almonds, and cassia oil. Additional suitable flavoring agents include, for example, vanilla, citrus oil (e.g., lemon, orange, grape, lime, grapefruit), citric acid, menthol, glycine, orange powder, cream, chocolate, mocha, spearmint, and cola. Suitable flavor essences include, for example, apple, apricot, banana, cherry, peach, pear, pineapple, plum, raspberry, strawberry, licorice flavor, orange vanilla flavor, creme de mint, cherry vanilla flavor, berry mix flavor, passion fruit flavor, mandarin orange flavor, bubble gum flavor, tropical punch flavor, juicy compound for grape, grape flavor, artificial grape flavor, grape bubble gum flavor, and tutti-frutti-flavor, and any combinations or mixtures thereof

Suitable anti-caking agents, e.g., suspending agents, include, but are not limited to, colloidal silicon dioxide, talc, tribasic calcium phosphate and any combinations or mixtures thereof. Those skilled in the art will understand how to select a suitable amount of anti-caking agent. For example, when preparing a suspension, the amount of anti-caking agent should be such that a firm, difficult to resuspend (beyond mild physical agitation) cake does not form under normal conditions of transportation and storage, but not an amount which causes gelation.

Suitable preservative for use in the present invention include, but are not limited to, parabens, benzyl alcohol, sodium benzoate, phenol, benzalkonium chloride, thimerosal, chlorobutanol, benzoic acid, sodium bisulfite, sodium proprionate and any combinations or mixtures thereof. As with the other optional excipients of the liquid dosage forms of the present invention, those skilled in the art will appreciate that the amount of preservative used in any particular dosage form is a function of, in large part, the exact preservative used, the pH of the dosage form, and the other components comprising the liquid dosage form.

Buffering agents suitable for use in the present invention include, but are not limited to, sodium phosphate dibasic anhydrous, sodium phosphate monobasic, and any combinations and mixtures thereof.

Additional excipients suitable for use in the present invention can be found in the Handbook of Pharmaceutical Excipients, 4th Edition 2003), the disclosure of which is hereby incorporated by reference.

In certain embodiments, the liquid dosage forms of the present invention may be prepared using a pre-manufactured vehicle such as, but not limited to, Ora-Plus^{®}, Ora-Blend^{®}; Ora-Blend^{®} SF; Suspendol-S™; Ora-Sweet^{®}; Ora-Sweet^{®} SF (all manufactured by Paddock Laboratories, Inc.) and the like.

In addition to the above-mentioned excipients, representative excipients and processes for preparation of liquid dosage forms are known in the art. See, e.g. Introduction to Pharmaceutical Dosage Fonns, 1985, Ansel, H. C., Lea and Febiger, Philadelphia, Pa.; Remington's Pharmaceutical Sciences, 1995, Mack Publ. Co., Easton, Pa., which are hereby incorporated by reference in their entireties.

### Detailed Description of the Preferred Embodiments

### Examples

The following examples illustrate various aspects of the present invention. They are not to be construed to limit the claims in any manner whatsoever.

### Example 1

### Single Dose Study

A single-center, randomized, double-blind, dose-rising study to determine the safety, pharmacokinetics, and pharmacodynamics of single doses of 1-(3-chloro-5-{[4-(4-chlorothiophen-2-yl)-5-(4-cyclohexylpiperazin-1-yl)thiazol-2-yl] carbamoyl} pyridin-2-yl)piperadine-4-carboxylic acid (Formula I) in normal healthy volunteers was performed. The starting dose of the drug of Formula I was 1 mg (expressed as free base). Successive dose cohorts received 3, 10, 20, 50, 75 and 100 mg. The drug substance in the form of the monomaleate salt was suspended in Ora-Plus® (purified water, microcrystalline cellulose, sodium carboxymethylcellulose, xanthan gum, citric acid, sodium phosphate, simethicone, potassium sorbate and methylparaben), a commercially available oral suspending vehicle manufactured by Paddock Laboratories, Inc. Minneapolis, MN. The Ora-Plus® was diluted with purified water, USP in a 1:1 suspension before being used to suspend the drug substance powder in preparation for oral administration. Prior to administration, 30 mL of the Ora-Plus®/ water vehicle was added to each bottle, shaken and then consumed by the subject for each of the 3, 10, 50 and 75mg dose cohorts. This was followed by an additional 30 mL of vehicle that was added to the bottle as a wash solution, shaken and then consumed by the subject. For the 20mg and 100mg dose cohorts, 60 mL of the Ora-Plus®/water vehicle was added to two (2) separate bottles containing the appropriate dose of drug (30 mL each bottle), shaken then consumed by the subjects. This was followed by an additional 60mL of vehicle that was added as 30 ml to each bottle as a wash solution, shaken and then consumed by the subject.

A minimum of 9 subjects (male and female healthy volunteers) were enrolled in each cohort. The active group within each cohort included male and female volunteers. Three subjects per cohort received placebo. Each successive dose cohort was treated no sooner than 10 days after the previous group had received the study drug. Dose escalation in succeeding groups did not proceed until all safety and pharmacodynamic parameters from Day 7 were reviewed by the Investigator and Sponsor. Each subject received only 1 dose of Formula I. Intra-subject dose escalation and treating previously treated subjects in any cohort expansions were not allowed. All subjects were followed until there platelet count returned to normal levels (defined as + 20% of baseline).

Dose escalation was stopped if the PD limiting dose was reached or at the dose at which a dose limiting toxicity (DLT) was observed. The pharmacodynamic limit (PD limit) is defined as the dose at which there was a 50% increase in platelet count relative to the baseline value of 5 in the 6 actively treated subjects. The pharmacodynamic limiting dose (if reached), was repeated by running an additional cohort of 9 subjects (3 placebo and 6 active) at the dose level of the observed PD limit.

Each subject was observed at regular (30 minute) intervals during the first 4 hours after study drug administration for close safety monitoring. Pharmacokinetic sampling was taken. Pharmacodynamic evaluations of platelet counts and other hematological parameters were performed from study drug administration (Day 1) until 7 days after dosing. Outpatient follow-up for safety evaluation occurred until 14 days post-treatment for all subjects and longer for subjects requiring additional follow-up. For the repeat PD dose cohort or 100mg dose cohort, the evaluations of platelet counts and other hematological parameters were performed each day from Day 1 through 14 and then every other day up to Day 28. If the PD dose limit was not reached at a dose of 75mg, platelet count evaluations as outlined for the PD limiting dose were performed on the 100mg dose cohort.

### Results

The resulting preliminary mean pharmacokinetic values calculated from Example 1 are shown in Table 1 below:

**Table 1**

| **Single-Dose PK** | | | |
|---|---|---|---|
| **Dose (mg)** | **AUC (ng*hr/mL)** | **Cmax (ng/mL)** | **T1/2 (hr)** |
| 1 | 161 | 5.15 | 18.9 |
| 3 | 477 | 16.0 | 17.5 |
| 10 | 2108 | 63.6 | 18.0 |
| 20 | 4018 | 146 | 12.9 |
| 50 | 9185 | 294 | 14.8 |
| 75 | 12567 | 446 | 15.3 |
| 100 | 10499 | 365 | 15.8 |

The final mean pharmacokinetic parameters calculated from Example 1 are shown in Table 2 below:

**Table 2**

| **Single-Dose PK** | | | | |
|---|---|---|---|---|
| **Dose (mg)** | **AUC₀₋ₗₐₛₜ (ng*hr/mL)** | **AUC_{0-inf.} (ng*hr/mL)** | **Cmax (ng/mL)** | **T1/2 (hr)** |
| 1 | 130.16 | 183.19 | 5.67 | 21.49 |
| 3 | 400.51 | 534.36 | 17.05 | 23.57 |
| 10 | 1645.15 | 2378.25 | 69.08 | 21.77 |
| 20 | 3597.72 | 4439.89 | 168.75 | 18.90 |
| 50 | 6879.32 | 8477.58 | 311.50 | 19.00 |
| 75 | 10824.65 | 13457.17 | 473.67 | 18.81 |
| 100 | 10863.54 | 11077.45 | 388.00 | 18.00 |

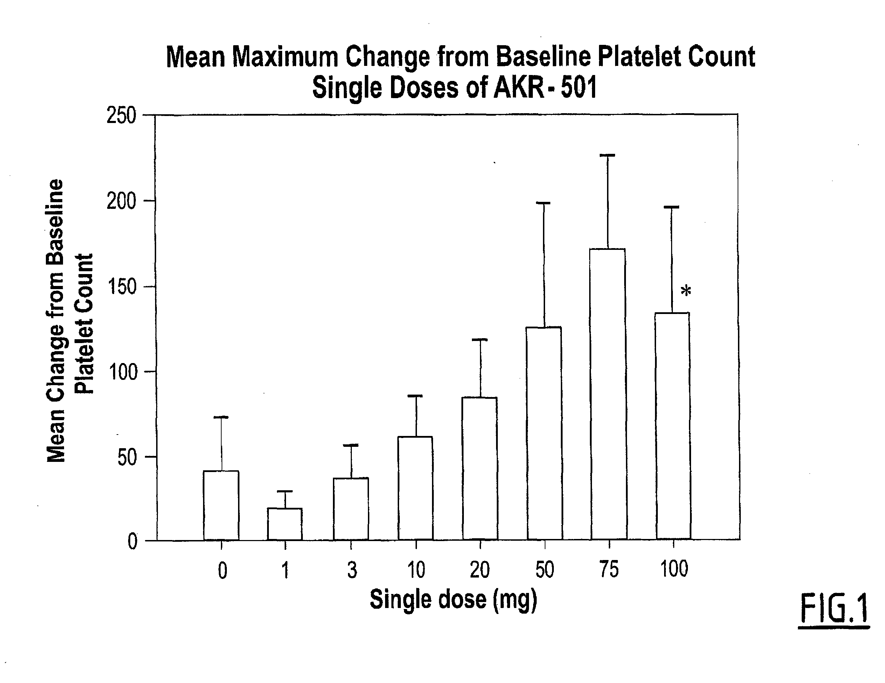

The change in platelet concentration resulting from Example 1, as measured in percent of baseline, is shown in Figure 1.

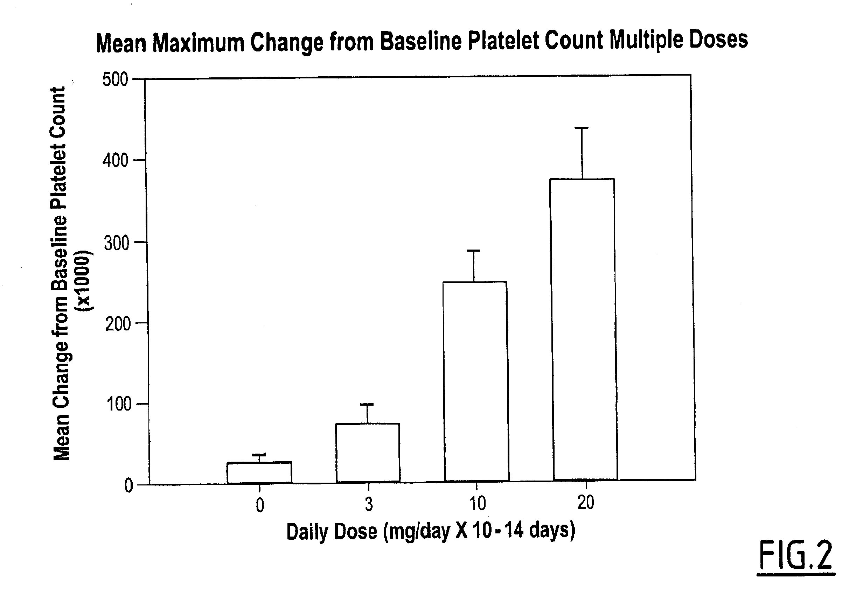

The mean maximum change from baseline platelet count from Example 1 is shown in Figure 2.

Pharmacokinetics were linear with consistently good absorption, a 2-4 hour lag time, and a serum elimination half-life of the drug was independent of the dose and estimated to be around 18 to 24 hours.

The Cmax and AUC increased proportionally up to the 75mg dose. Slightly lower than expected increases in drug exposure were evident following a single dose of 100mg and were likely due to a lower exposure in one outlying subject.

Pharmacokinetic/pharmacodynamic analysis showed a highly significant dose and concentration related effect on platelet count. The magnitude of the increase in platelet counts observed corresponded with the dose of drug administered and statistically significant increases in platelet counts were evident following a single dose of≥ 10mg of drug. Changes in platelet counts were evident as early as 3 to 5 days following a single dose of drug (10mg to 100mg) and the highest observed changes in platelet counts were observed by approximately 6 to 10 days. The FDA mandated PD limit (5 subjects with > 50% increase in platelet count) was reached at the 100mg dose.

A dose-concentration-response relationship was present for drug and changes in platelet count as increasing drug concentrations and exposure resulted in linear increases in platelet counts over baseline.

The drug was well tolerated with no significant drug-related clinical or laboratory adverse experiences up to a dose of 100mg.

### Example 2

### Multi-Dose Study

A double-blind, placebo-controlled, dose rising study was performed in healthy male and female volunteers. Each dose cohort received either placebo or active treatment as an oral suspension once daily for fourteen (14) consecutive days. The dose of study drug was prepared and administered as described in Example 1 above. The doses administered in this multiple dose study were based on the safety and tolerability assessment of the doses administered in Example 1. The dose level in this study always remained at least one dose level lower than the next highest dose level at which dose safety and tolerability were demonstrated in Example 1.

Based on the results of Example 1, the starting dose was 3 mg. Based on the safety and tolerability results from Example 1, the dose was scheduled to be escalated to 10, 20, 50 and 100 mg per day for 14 consecutive days. Successive dose cohorts were not treated until the previous cohort completed 14 days of treatment and the safety follow-up visit on Day 21. Dose escalation from 50mg to 100mg did not proceed as the study was discontinued after enrollment of the 20mg cohort due to the fact that all subjects had reached a PD limit platelelt count (≥ 500,000mm³).

### Results

The mean pharmacokinetic values calculated from Example 1 are shown in Table 3 below:

**Table 3**

| **Parameters** | **Study Day** | **3mg** | **10mg** | **20mg*** |
|---|---|---|---|---|
| Cₘₐₓ (ng/mL) | 1 | 14.8 (23.5%) | 53.2 (27.6) | 121.4 (37%) |
| Tₘₐₓ (hr)^{a} | 1 | 6 (3-6) | 6 (6-6) | 6 (3-6) |
| AUC₀₋ₗₐₛₜ (ng*hr/mL) | 1 | 235.4 (21.4%) | 840.8 (25.7%) | 1956.7(37.8%) |
| C_{max,ss} (ng/mL) | 14 | 25.6 (13.7%) | 94.1 (15.8%) | 204.8 (44.7%) |
| T_{max,ss} (hr)^{a} | 14 | 6 (3-12) | 4.5 (2.5-6) | 6 (3-6) |
| AUC₀₋ₗₐₛₜ (ng*hr/mL) | 14 | 659.4 (19.4%) | 2364.7 (18.5%) | 3610.0 (51.7%) |
| AUC_{0-inf.} (ng*hr/mL) | 14 | 804.3 (24.5%) | 2868.0 (21.6%) | 7148.1 (76.9%) |
| T_{1/2} (hr) | 14 | 18.2. (21.6%) | 18.1 (10.8%) | 20.8 (34.5%) |

| | | | | |
|---|---|---|---|---|
| * Simulated data was used for 20mg at steady-state. a. median (range) | | | | |

The change in platelet concentration from Example 2 is shown in Figure 2.

The drug was well tolerated with no significant drug-related clinical or laboratory adverse experiences up to a dose of 20mg daily for ten (10) to fourteen (14) days.

The Cmax and AUC increased proportionally with respect to dose following single and repeat administration.

The t_{1/2} of the drug was independent of dose and was estimated to be approximately 18 to 21 hours.

The accumulation of drug following repeat doses can be predicted from the single dose data of Example 1.

Pharmacokinetic/Pharmacodynammic analysis showed highly significant dose and concentration related effects on platelet count. The magnitude of the increases in platelet counts increased with the dose of drug administered. Statistically significant increases in platelet counts were evident following repeat administration of 10mg and 20mg. Changes in the platelet counts were evident as early as 3 to 5 days following dosing of drug and maximum changes in platelet count increases were dependent on dose, concentration, and duration of treatment. The original PD limit of five (5) subjects with > 50% increase in platelet count was reached in the 10mg cohort. The FDA reviewed all of the safety data after the 10mg cohort and approved escalation to the 20mg cohort with a revised PD limit of 5 subjects with > 500,000 platelets. A revised PD limit occurred in all six (6) subjects by day 10-11 in the 20mg cohort.

A dose-concentration-response relationship was present for drug and changes in platelet count as increasing drug concentrations and exposure resulted in linear increases in platelet counts over baseline within the 3mg to 20mg dose range tested.

### Example 3

### Food Effect Study

An open label, randomized, three-way, crossover study to evaluate the pharmacokinetics, relative bioavailability, and safety of a 10mg oral dose of drug of Formula I administered to healthy male and female volunteers as an oral suspension or a tablet under fed and fasted conditions was conducted.

Eighteen subject were enrolled in the study.

The preliminary pharmacokinetic parameters are outlined in Table 4 below:

**Table 4**

| **Dosage Form** | **Cmax** | **Tmax** | **AUCₗₐₛₜ** | **AUC_{inf.}** | **T_{1/2}** |
|---|---|---|---|---|---|
| Susp. Fasted | 57.575 | 8.25 | 1833.63 | 1952.983 | 21.206 |
| Tablet Fasted | 39.68 | 6.8 | 1226.767 | 1308.567 | 20.804 |
| Tablet Fed | 45.106 | 7.176 | 1346.311 | 1451.851 | 21.277 |

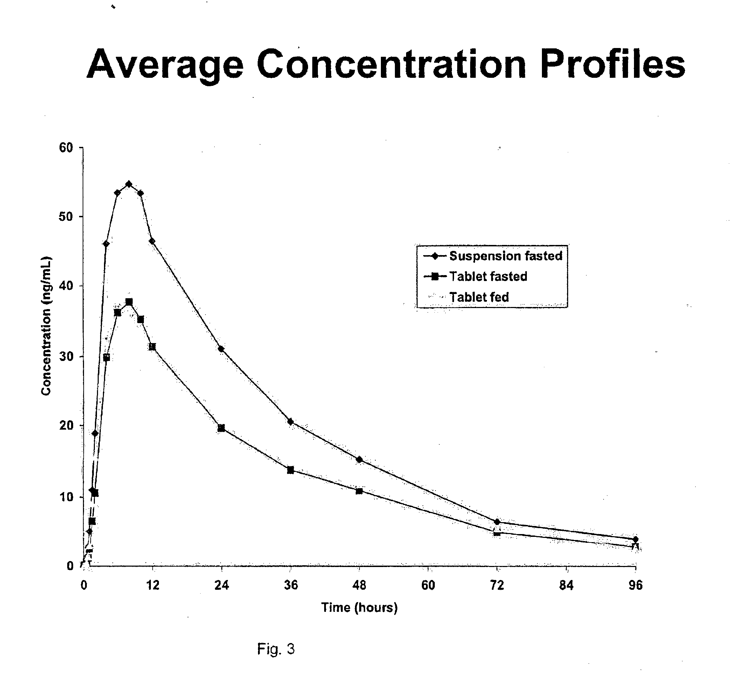

The bioavailability of the tablet was about 67% and there did not appear to be a food effect for the tablet as the difference in pharmacokinetics for the fed and fasted tablet treatments is approximately 10%. Figure 3 shows the average concentration profiles for the 10mg oral suspension (fasted), and 10mg tablets (fed and fasted).

## Claims

1. An immediate release oral dosage formulation comprising a pharmaceutically acceptable excipient and 10 to 75 mg of the drug of Formula I or a pharmaceutically acceptable salt thereof, the dosage form providing a mean maximum plasma concentration (Cₘₐₓ) of from 3 ng/ml to 8 ng/ml for each 1 mg of the drug included in the dosage form, after a single dose administration to a human subject:

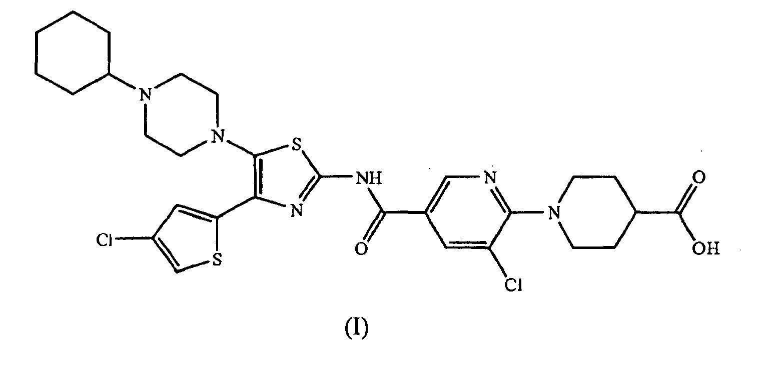

2. The oral pharmaceutical dosage form of claim 1, comprising 10, 20, 50 or 75 mg of the drug of Formula I or a pharmaceutically acceptable salt thereof.

3. The oral pharmaceutical dosage form of claim 1, wherein the pharmaceutically acceptable salt is a mineral acid, an organic acid, a salt with an inorganic base, a salt with an organic base, or ammonium salts.

4. The oral pharmaceutical dosage form of claim 3, wherein the pharmaceutically acceptable salt is a hydrochloric acid, hydrobromic acid, hydroiodic acid, sulfuric acid, nitric acid, phosphoric acid, formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, p-toluenesulfonid acid, aspartic acid, glutamic acid, sodium, potassium, magnesium, calcium, methylamine, ethylamine, ethanolamine, lysine, or ornithine.

5. The oral pharmaceutical dosage form of claims I to 4, in the form of a tablet, soft or hard gelatin capsule, solution or suspension.

## Patentansprüche

1. Orale Darreichungsform zur sofortigen Freisetzung, umfassend einen pharmazeutisch akzeptablen Exzipienten und 10 bis 75 mg des Wirkstoffs der Formel Formel (I) oder eines pharmazeutisch akzeptablen Salzes davon, wobei die Darreichungsform eine mittlere maximale Plasmakonzentration (Cₘₐₓ) von 3 ng/ml bis 8 ng/ml je 1 mg des in der Darreichungsform enthaltenen Wirkstoffs nach Verabreichung einer einzelnen Dosis an ein menschliches Subjekt bereitstellt.

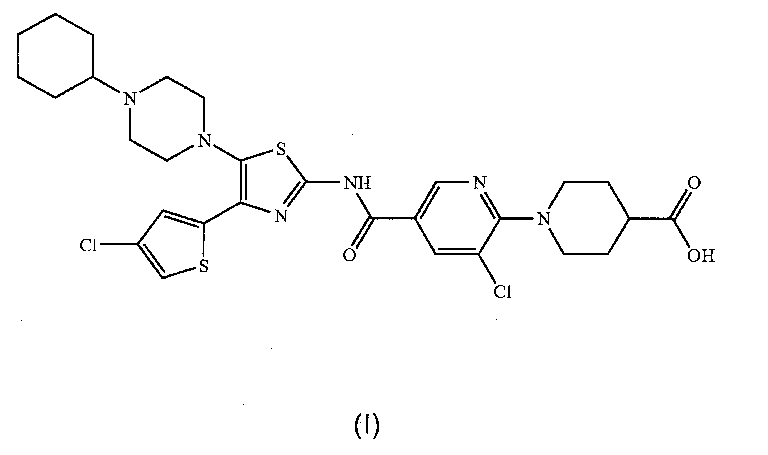

2. Orale pharmazeutische Darreichungsform gemäß Anspruch 1, umfassend 10, 20, 50 oder 75 mg des Wirkstoffs der Formel I oder eines pharmazeutisch akzeptablen Salzes davon.

3. Orale pharmazeutische Darreichungsform gemäß Anspruch 1, wobei das pharmazeutisch akzeptable Salz eine Mineralsäure, eine organische Säure, ein Salz mit einer anorganischen Base, ein Salz mit einer organischen Base oder Ammoniumsalze ist.

4. Orale pharmazeutische Darreichungsform gemäß Anspruch 3, wobei das pharmazeutisch akzeptable Salz Salzsäure, Bromwasserstoffsäure, Iodwasserstoffsäure, Schwefelsäure, Salpetersäure, Phosphorsäure, Ameisensäure, Essigsäure, Propionsäure, Oxalsäure, Malonsäure, Bernsteinsäure, Fumarsäure, Maleinsäure, Milchsäure, Äpfelsäure, Weinsäure, Zitronensäure, Methansulfonsäure, Ethansulfonsäure, p-Toluolsulfonsäure, Asparaginsäure, Glutaminsäure, Natrium, Kalium, Magnesium, Calcium, Methylamin, Ethylamin, Ethanolamin, Lysin oder Ornithin ist.

5. Orale pharmazeutische Darreichungsform gemäß Ansprüchen 1 bis 4 in Form einer Tablette, einer Weich- oder Hartgelatinekapsel, einer Lösung oder einer Suspension.

## Revendications

1. Formulation posologique orale à libération immédiate comprenant un excipient pharmaceutiquement acceptable et 10 à 75 mg du médicament de formule I ou d'un sel pharmaceutiquement acceptable de celui-ci, la forme posologique fournissant une concentration plasmatique maximale moyenne (Cₘₐₓ) de 3 ng/ml à 8 ng/ml pour chaque 1 mg du médicament inclus dans la forme posologique, après l'administration d'une seule dose à un sujet humain :

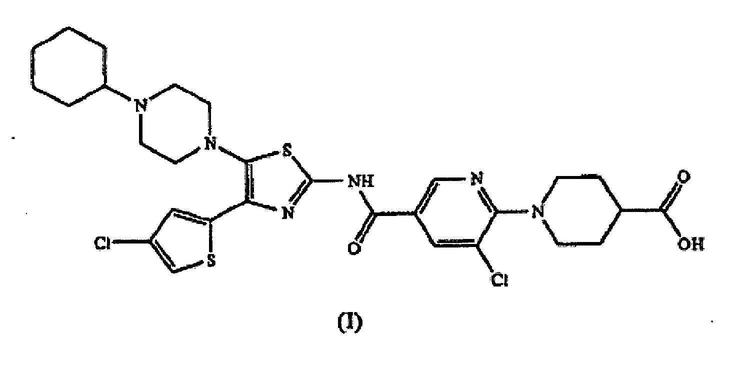

2. Forme posologique pharmaceutique orale selon la revendication 1, comprenant 10, 20, 50 ou 75 mg du médicament de formule I ou d'un sel pharmaceutiquement acceptable de celui-ci.

3. Forme posologique pharmaceutique orale selon la revendication 1, dans laquelle le sel pharmaceutiquement acceptable est un acide minéral, un acide organique, un sel avec une base inorganique, un sel avec une base organique, ou des sels d'ammonium.

4. Forme posologique pharmaceutique orale selon la revendication 3, dans laquelle le sel pharmaceutiquement acceptable est un acide chlorhydrique, acide bromhydrique, acide iodhydrique, acide sulfurique, acide nitrique, acide phosphorique, acide formique, acide acétique, acide propionique, acide oxalique, acide malonique, acide succinique, acide fumarique, acide maléique, acide lactique, acide malique, acide tartrique, acide citrique, acide méthane-sulfonique, acide éthane-sulfonique, acide p-toluène sulfonique, acide aspartique, acide glutamique, sodium, potassium, magnésium, calcium, méthylamine, éthylamine, éthanolamine, lysine, ou ornithine.

5. Forme posologique pharmaceutique orale selon les revendications 1 à 4, sous la forme d'un comprimé, d'une gélule de gélatine molle ou dure, d'une solution ou d'une suspension.
